# EUROPEAN PATENT SPECIFICATION

(11) **EP 4 143 240 B1**
(45) Date of publication and mention of the grant of the patent: **20.09.2023**
(21) Application number: 21721641.5
(22) Date of filing: 28.04.2021
(51) Int. Cl.: C08B 37/08, C08B 37/02, C08B 37/00

(54) **NANOPOROUS MICROSPONGE PARTICLES (NMP) OF BIOCOMPATIBLE POLYMERS AS UNIVERSAL CARRIERS FOR BIOMOLECULES DELIVERY**
NANOPORÖSE MIKROPARTIKEL (NMP) AUS BIOKOMPATIBLEN POLYMEREN ALS UNIVERSELLE TRÄGER ZUR FREISETZUNG VON BIOMOLEKÜLEN
PARTICULES DE NANOÉPONGE NANOPOREUSE (NMP) DE POLYMÈRES BIOCOMPATIBLES EN TANT QUE SUPPORTS UNIVERSELS POUR L'ADMINISTRATION DE BIOMOLÉCULES

(30) Priority: 28.04.2020 EP 20171927
(43) Date of publication of application: 08.03.2023
(73) Proprietor: Nanofaber S.r.l., 00154 Roma (IT)
(72) Inventor: CAVALIERI, Francesca, 00154 Roma (IT); RINALDI, Antonio, 00154 Roma (IT)
(74) Representative: Studio Torta S.p.A.
(86) International application number: PCT/IB2021/053536
(87) International publication number: WO 2021/220195

(56) References cited:
- EP-B1- 3 294 778
- CASO MARIA FEDERICA ET AL: "Nanoporous Microsponge Particles (NMP) of Polysaccharides as Universal Carriers for Biomolecules Delivery", NANOMATERIALS, vol. 10, no. 6, 31 May 2020 (2020-05-31), page 1075, XP055824881, ISSN: 2079-4991, DOI: 10.3390/nano10061075

## Description

### CROSS-REFERENCE TO RELATED APPLICATIONS

This patent application claims priority from European patent application no. 20171927.5 filed on 28/04/2020.

### TECHNICAL FIELD

The present invention concerns a method to produce nanoporous microsponge particles useful as universal carriers for biomolecules delivery.

### BACKGROUND ART

Organic-inorganic nanoporous multifaceted structures, such as micro-nanoflowers, microsponges, nanoclews, and nanococoons are emerging materials that have proven useful in applications spanning catalysis, sensing and drug delivery, owing to their porous structure. The fabrication of these systems typically requires the addition of inorganic component, such as a metal salt (e.g., composed of copper, calcium, or manganese phosphate), to biomacromolecules. This approach involves the functional groups of the biomacromolecules, which provide binding sites for the nucleation and subsequent growth of metal phosphate crystals, leading to formation of nanostructured phosphate-based microparticles, with a high surface-to-volume ratio and signature porous structures.

As it may be obvious to a skilled man of the art, the above synthesis method can have some limitations regarding the use of toxic elements or extreme harsh conditions.

Nanoporous microparticles have primarily been used for the immobilization of enzymes with preserved catalytic performances, for intracellular imaging, and for protein and drug delivery. In particular, nanoporous microparticles hold greater potential for regenerative medicine and pharma-cosmetics as a drug delivery system for synthetic drugs and biomolecules in general (e.g. peptides, growth factors, mRNA, etc.). In this regards, the nanoporous microparticles must exhibit high levels of safety and biomolecules loading capacity.

The need is felt to have a nanoporous microparticles that represent a highly efficient platform for biomolecules delivery and that can be synthetized by methods that do not have the limitations of conventional methods.

### OBJECT OF THE INVENTION

The object of the present invention is a method to produce nanoporous microsponge particles useful as carriers for drug delivery; said method comprising a cross-linking/precipitation step wherein a cross-linking agent reacts with hyaluronic acid having nucleophile functional groups which react with said cross-linking agent; said a cross-linking agent being prepared by a reaction between 1,1'-carbonyldiimidazole (CDI) and a diamine compound; said method being characterized in that said hyaluronic acid has an average molecular weight ranging from 10KDa to 200KDa.

The average molecular weights given in the description are calculated by the method in the international standard ISO 11344.

Here and below the term "drug" refers to compound involved in a medical treatment, regardless of its molecular weight and its charge.

As it will be shown below, the molecular weight of the biocompatible polymers turns out to be a determining feature for giving the nanoporous microsponge particles the sought properties (drugs delivery carriers). In fact, it has been experimentally proved that when biocompatible polymers have molecular weight higher than the claimed range, the produced nanoporous microsponge particles are characterized by a small swelling ratio. Differently, when biocompatible polymer have molecular weight comprised in the claimed range, the produced nanoporous microsponge particles are characterized by a high swelling ratio.

The swelling ratio of a nanoporous microsponge particle indicates a high capability to adsorb aqueous solution. In other words, the swelling capability of nanoporous microsponge particles is a telling ex-post measurement of capillary suction of a nanoporous microsponge particle and, accordingly, is very relevant for drug (the solute in an aqueous solution) upload.

In particular, it is worthy to underline that the prior art (EP3294778B1 - [0026]) stated that, by using a cross-linking agent according to the invention (a cross-linking agent prepared by causing 1,1'-carbonyldiimidazole (CDI) to react with a diamine compound), it is not possible to synthetize nanoporous microsponge particles starting from a polysaccharide having a molecular weight lower than 250KDa.

Differently, the inventors of the present invention demonstrated that it is possible to prepare nanoporous microsponge particles starting from a polymer having a molecular weight much lower than 250KDa and, above all, that such nanoporous microsponge particles are extremely suitable for use as carriers for drug delivery.

Preferably, said hyaluronic acid has an average molecular weight ranging from 20KDa to 150KDa.

Preferably, said hyaluronic acid is used at a concentration ranging from 0.5 to 1.5, more preferably from 0.8 to 1.0 g/dL, and the cross-linking agent is used at a concentration ranging from 0.05 to 0.15 M, more preferably from 0.05 to 0.10 M.

Preferably, said 1,1'-carbonyldiimidazole (CDI) reacts with a diamine compound in water or in dimethyl sulfoxide.

Preferably, said diamine compound is Cystamine dihydrochloride.

A further object of the present invention consists of nanoporous microsponge particles prepared by the method of the present invention and characterized by having an average diameter ranging from 1.0 to 15.0 µm and an average size of pores ranging from 10 to 400 nm.

Preferably, the nanoporous microsponge particles have a swelling ratio higher than 1.5.

Preferably, the nanoporous microsponge particles have an average diameter ranging from 3.0 to 8.0 µm, an average pore size ranging from 100 to 300 nm.

Herein and below for "swelling ratio" it is meant sD/dD; where sD is the diameter of the swelled nanoporous microsponge particle after immersion in an aqueous solution and dD is the diameter of the dry nanoporous microsponge particle before immersion in an aqueous solution.

Another further object of the present invention consists of use of nanoporous microsponge particles according to the present invention as carriers for drug delivery.

Preferably, the drugs for microsponge of the invention are comprised in the group consisting of: Lipids (e.g. linolenic acid); Proteins (e.g. lysozime, albumin, and therapeutic proteins defined, including α-1-proteinase inhibitor, lactase, pancreatic enzymes, salmon calcitonin, becaplermin, trypsin, collagenase, human deoxy-ribonuclease I, dornase-α, hyaluronidase, papain, etc.]); growth factors; cross-linked hyaluronic acid; anti-VEGF; oncotherapeutics (e.g. methotrexate, aflibercept, doxorubicin, cisplatin, 5-fluroacil); insulin, semaglutide, glucagon-like peptide 1 receptor agonists (GLP-1); NSAID; cortico-steroids (e.g. dexamethasone); disease modifying drugs* (e.g. conventional drugs, such as ciclosporin, cyclophosphamide, hydroxychloroquine, leflunomide, methotrexate, mycophenolate, sulfasalazine; or biological or anti-TNF therapies, such as abatacept, rituximab, tocilizumab, adalimumab, certolizumab pegol, etanercept, golimumab, infliximab;* For the specific application of rheumatoid arthritis they are also known as DMARDs; monoclonal antibodies, RNA, DNA and peptides; vaccine adjuvants and vectors; exosomes; antibiotics; oppioids and analgesics; nanoparticles.

Preferably, nanoporous microsponge particles according to the present invention are used as a medicament.

The present invention refers to a technique to prepare nanoporous microsponge particles as platform for drug delivery. In the experimental part below it is reported the successful loading of nanoporous microsponge particles with two proteins of scientific and pharmacological relevance, i.e. bovine serum albumin (BSA) and lysozyme. Because these proteins have different molecular weight and isoelectric point, they represent significant case studies to assess the loading and release capacity of the polysaccharides nanoporous microsponge particles (NMPs) .

In particular, the nanoporous microsponge particles of the invention provide a platform for delivery a variety of biomacromolecules, such as proteins, lipids and nucleic acids which can hardly pass through the various biological barriers without the assistance of a carrier. Specifically, polysaccharides are widely regarded and studied as potential carrier materials for site specific drug delivery because of their non-toxic, biocompatible and biodegradable properties. These systems can be used to provide targeted delivery of drugs (at cellular or tissue level) and, for example, to improve their ophthalmic or oral bioavailability, as well as in the intra-articular therapy of chronic rheumatic disease.

In addition, nanoporous microsponge particles hold greater potential for regenerative medicine and pharma-cosmetics as a drug delivery system for synthetic drugs and biomacromolecules in general (e.g. peptides, growth factors, mRNA, etc.).

It is important to stress that the new co-precipitation method of the invention uses an organic molecule, i.e. cross-linking agent, to obtain nanoporous microsponge particles without resorting to metal ions. During the microparticles preparation, the biocompatible polymer chains present in the reaction mixture mediate the nucleation and growth of cross-linking agent crystals leading to nanoporous microsponge particles formation with signature structural and functional properties. Compared to the inorganic salts used in the synthesis of prior art, the developed cross-linker offers several advantages including reactivity with amine hydroxyl and carboxyl groups and redox responsive behaviour.

### BRIEF DESCRIPTION OF THE DRAWINGS

For a better understanding of the invention, real examples - yet purely illustrative and non-limiting - are provided in the following with the aid of accompanying figures, wherein:
Figure 1 shows confocal microscopy and scanning electron microscopy imagines of the synthetized nanoporous microsponge particles of the invention;
Figure 2 shows four graphs relating to size distribution synthetized nanoporous microsponge particles of the invention in dry and swollen condition;
Figure 3 shows two graphs relating to adsorption of proteins by nanoporous microsponge particles of the invention;
Figure 4 shows two graphs relating to release of proteins by nanoporous microsponge particles of the invention;
Figure 5 shows four graphs relating to pH dependence of adsorption and release of proteins by nanoporous microsponge particles of the invention;
Figure 6 shows confocal microscopy imagines of proteins adsorbed by nanoporous microsponge particles of the invention at different pH;
Figure 7 shows two graphs relating to cytotoxicity in vitro of nanoporous microsponge particles of the invention vs fibroblasts; and
Figure 8 shows a graph relating to cellular adsorption of nanoporous microsponge particles of the invention.

### Materials

Cystamine dihydrochloride (CYS), 1.1 Carbonyldiimidazole (CDI) and sodium alginate (Alginate, MW 140 kDa), dextran sulfate sodium salt (Dextran, MW 40 kDa), carboxymethyl-dextran sodium salt (CM-Dextran - MW 70 kDa), hyaluronic acid (Hyaluronic Acid low MW HA, 70 kDa), Rhodamine B isothiocyanate (RITC), DL-Dithiothreitol 99% (DTT), Bovine Serum Albumin, 96% (BSA), dimethyl sulfoxide (DMSO), lysozyme from chicken egg white, 90% were purchased from Sigma-Aldrich (St. Louis, MO, USA)

### Synthesis and characterization of the cross-linking solutions

The cross-linker (CL) solution was obtained from CDI and CYS dissolved in DMSO in a 3:2 weight ratio. The reaction proceeded overnight without stirring. Alternatively, another cross-linker solution was obtained by dissolving CDI and CYS in MilliQ water to obtain a precipitate. Subsequently the precipitate was dissolved in MiliQ at pH 4 by adding hydrochloric acid 2M (as needed). NMR spectra were recorded in deuterated DMSO to characterize the cross-linker (NMR spectrometer operating at 400 MHz, Bruker DRX, Bruker AVANCE).

### Synthesis and characterization of nanoporous microsponge particles (NMPs)

Four polysaccharides (dextran, CM-Dextran, hyaluronic acid, and alginate) were used as biocompatible polymers. Hyaluronic acid is according to the invention. Dextran, CM-dextran and alginate are reference examples. In fact, such polysaccharides represent biocompatible polymers having nucleophile functional groups (in particular - OH and -CO2H) able to react with the cross-linking agent. One-milliliter samples of 1 % w/v solution of were individually added to cross-linker solutions. The reaction proceeded overnight without stirring. The resulting NMP particles were centrifuged and washed with MilliQ water three times. The yields of the reactions were calculated as follows: the NMP were formed using fluorescently labelled polysaccharide and the UV-vis absorbance "ab" (Cary 100 spectrophotometer) of the polysaccharide solution was measured before NMP microparticles formation. Next the suspension was centrifuged and the supernatant analysed by measuring the UV-vis absorbance "aa". The yield was estimated by comparing the two absorbances as "(ab- aa)/ab 100%".

Particles were characterized with a Zeiss fluorescence microscope (Axio Scope.A1) equipped with a mercury-vapour short-arc-lamp HB = 50 W/AC at 560 nm emission wavelength and 63 × objective, a confocal microscope (FluoView, 1000 LSCM system, Olympus, Tokyo, Japan), and a SEM (model Leo Supra-35, Carl Zeiss Microscopy GmbH, Jena, Germany). Prior to SEM observation, the samples were vacuum-dried. The above procedure applied identically for polymer derivatives labelled with RITC (ref. 2.4) . SEM and confocal microscopy images were used to measure the diameters of NMP in vacuum-dry and wet conditions respectively. The comparison between the two measurements was used to estimate the swelling ratio. Pore size distribution of NMP was evaluated by SEM micrographs of vacuum-dried NMPs. Particle diameter and pore size measurements were carried out on micrographs of about 100 different microparticles using Image-J (NIH software, USA).

### Preparation of polysaccharide fluorescently labelled with Rhodamine B isothiocyanate

For NMPs used in fluorescence assays, the standard preparation of polysaccharide in Section 2.3 was modified by dissolving Rhodamine B isothiocyanate (RITC) into DMSO and by adding it to the standard 1% solution of polysaccharide. The resulting solution was stirred for a few hours at room temperature. To remove residual non-conjugated dyes, the samples were dialyzed using a dialysis membrane 12-14 000 Daltons (Medicell International, London), the degree of functionalization were calculated using the absorbance measured for pre-weighted samples of stained polysaccharide and Beer-Lambert-Bouguer law.

### Degradation Kinetics of NMP

The degradation study was carried up over 24 hours, when the degradation appeared complete. An aliquot of 1 mL was used. At variable time intervals, the suspension was centrifuged, and the supernatant was analysed by UV-vis spectra. The supernatant contains the microparticles components after disassembly including the fluorescently labelled polysaccharides.

### Protein adsorption and release in particles

Lysozyme and BSA were labelled as follows: Rhodamine B isothiocyanate (RITC) was dissolved into DMSO and 10 uL were added to 1 mg/mL protein solution. The resulting solution was stirred for a few hours at room temperature. To remove residual non-conjugated dyes, the samples were dialyzed using a dialysis membrane 12-14 000 Daltons (Medicell International, London), the degree of functionalization was calculated as described above. The loading of the proteins was performed as follows: 1.75 mg of RITC-lysozyme and 1.75 mg of RITC-BSA were dissolved in 1 mL PBS at pH 7 (precisely pH 7.4). Next, a suspension of NMP containing 1 mg of polysaccharides was added to the protein solutions. At variable intervals, the resulting suspensions were centrifuged, and the supernatants were analysed by UV-vis spectroscopy (peaks at 560 nm for RITC). The plateau in the adsorption curve was obtained after 24 hours incubation up to 250 h and the loading capacity was calculated using the maximum absorption values in the adsorption curves (@24h plateau).

To study dependence of protein loading and unloading in NMPs as a function of pH solution, additional 1.75 mg of RITC-lysozyme (lysozyme from chicken egg white, Sigma-Aldrich, 90%) and 1.75 mg of RITC-BSA (bovine serum albumin, Sigma-Aldrich, 96%) were solved in 1 ml of PBS at pH 5 and 11 to obtain 4 more solutions. Each solution was monitored during 24 hours at room temperature to test the stability of proteins at the different pH. d-NMPs and w-NMPs (polysaccharides concentration in both: 1.5 g/l) obtained from CL dissolved in DMSO and in H2O respectively were added the NMPs, yielding 48 different samples for each combination of {protein (2), polysaccharide (4), CL protocol(2), pH (3)}. At variable intervals, the resulting suspensions were centrifuged, and the supernatants were analysed with UV-vis spectra (peaks at 560 nm for RITC) . The plateau of adsorption curve, that is the maximum of adsorption, was measured after 24 hours. The lysozyme and BSA adsorbed particles were centrifuged and suspended in 1 ml of PBS at pH 5, 7 and 11. The resulting suspensions were centrifuged at variable intervals and the supernatant was analyzed with UV-vis spectra. Every 24 hours fresh PBS was added. The plateau of release curve was obtained at 150 hours for calculation sake (but the study was carried out for 300 hours)

### Cell assays for cito-toxicity and adhesion/internalization of NMPs

Fibroblasts 3T3 were chosen as model cell line and cultured at 37°C in a 5% CO2 atmosphere, in DMEM and L-Glutamine supplemented with 10% bovine calf serum (Gibco). Cells were seeded at a density of 5 × 103 cells/ cm2 (on 24-well plates or cover slips) and were allowed to attach for 24 hours prior to the experiments until the population confluence reached approximately 80%. The NMPs were premixed with culture medium and incubated with 3T3 fibroblasts for different times and concentrations.

### Cell Viability

The trypan blue was used to determine the number of viable cells incubated with particles. It is based on the principle that live cells possess intact cell membranes that exclude certain dyes, such as trypan blue, whereas dead cells do not. The cells were cultured to 80% confluence in 24-well plates and each type of NMP was added to medium for 24 or 120 hours in individual wells. The "120h" time point was selected arbitrarily to show the impact of NMP on cell viability at prolonged incubation time. Cells cultured without NMPs were considered as controls. In each set of experiments, the polysaccharide concentration was considered. An additional control was performed adding the CL in complete medium without the polysaccharide. After incubation for 24 and 120 hours, the medium was removed and collected, and the cells were detached by 0.25 % Trypsin/EDTA solution. Following this step, the cell suspension was centrifuged, and the cell were stained with 0.4 % tryptan blue (Sigma-Aldrich). The stained cells were visualized with an inverted microscope (Leica, Germany). The dead and living cells were counted by a Burker-Türk counting chamber and the percentage of living cells was determined.

### Study of cell adhesion/internalization of NMPs by confocal microscopy

To investigate at the confocal microscope the possible internalization of NMP microsponges by 3T3 fibroblasts, NMPs were labeled with fluorescein-5-isothiocyanate (FITC). After culturing the cells for 24h, they were incubated in plates with FITC-labelled NMP particles (0.25 g/ml) in DMEM 10% serum for 4h, 24h or 48h. At the end of each time, the cells were washed with PBS to remove the excess/unbound NMPs and dead cells. The dead and living cells were counted by a Bürker-Türk counting chamber and the percentage of living cells was determined as an estimate of adherent cells after 4h, 24h or 48h incubation with NMPs. After incubation cells were fixed and stained with a red fluorescent marker wheat germ agglutinin (WGA) conjugated to Texas Red (10 ug/mL; Sigma-Aldrich) for 15 min at 37° C to label the cell, which selectively recognizes plasma and Golgi/ER membrane structures. Afterwards the cells were fixed with cold methanol and stained with Hoescht 33258 (1 gg/mL; Sigma-Aldrich), to visualize the nuclei. Such samples were observed by means of a confocal laser scanning microscopy (Olympus Fluoview 1000). Confocal X-Y, X-Z, Y-Z sections in max intensity projection mode (MIP modality) were taken to display three-dimensional images of MPs cell interaction.

### Results and Discussion

### - Two-ways parametric study on NMP synthesis vs. (i) polymer and (ii) cross-linker solvent -

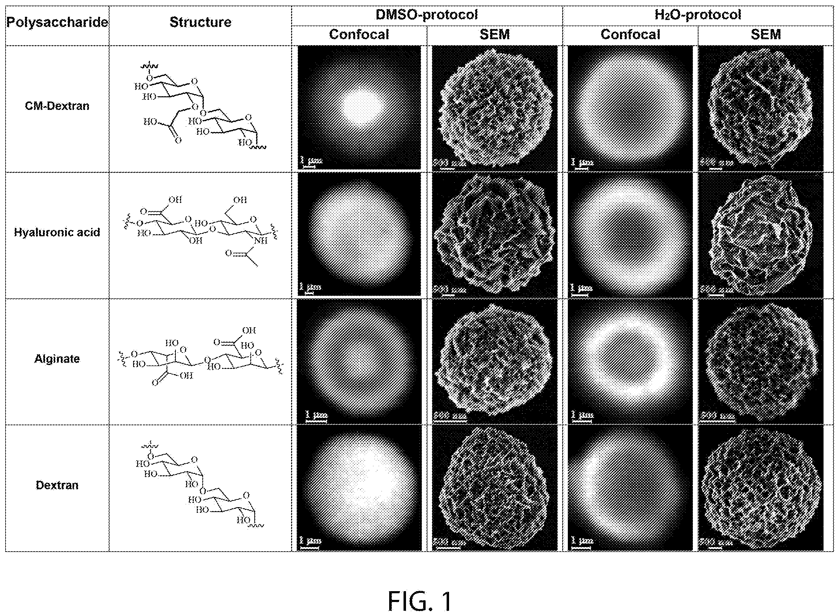

Eight batches (4X2) of nanoporous polysaccharide microsponges particles (NMP) were synthetized according to the standard one-pot self-precipitation/cross-linking method using four RITC-labelled polymers (i.e. alginate, hyaluronic acid, CM-dextran and dextran) and two alternative protocols based on solvent selection (i.e. DMSO vs. H2O route). The purpose was to investigate the effects on structure and morphology of the resulting NMP for a given pair polymer-solvent. The results are summarized in Figure 1, which displays for each batch the representative micrographies obtained via (i) confocal microscopy and (ii) scanning electron microscopy.

The latter techniques provide complementary information and, while SEM reveals the overall morphology (e.g. shape, dimension, surface roughness, porosity), the confocal microscopy clearly render an insight about the "structural" properties in terms of polymer density within the NMP. The morphology of the microsponges obtained in the DMSO (d-NMP) and in pure water (w-NMP) demonstrates the successful formation of NMP with all different polysaccharides and indicates that CL can also react with either neutral polysaccharides or negatively charged polysaccharides chain to form NMP. The cross-linker crystalline domains form the scaffold of the particles whereas the polymer chains act as a "glue" to bind the CL crystalline domains together.

Remarkably, the w-NMPs exhibit consistently a core shell structure with a less fluorescent core and a highly fluorescent corona. This indicates that the RITC-polysaccharides are predominately located on the periphery of the NMP. Conversely, the d-NMP synthetized via the DMSO route shows an even distribution of the fluorescent polysaccharides throughout the NMP particle for dextran and HA and a fluorescent core in the case of alginate and CM-dextran. These different structures can be ascribed to the different kinetic of crystallization of the CL in water and DMSO. The faster precipitation of the CL in water produces a crystalline core excluding the polysaccharides chains; subsequently the polysaccharides chains form a composite polysaccharides-CL porous outer corona. During the slow precipitation of CL in the case of DMSO solvent the polysaccharide chains remain embedded during the all process of growth of NMP. A peculiar onion-like structure was observed for alginate-based d-NMP with an inner layer composed primarily of CL.

The general NMP formation process entails that the carbonyl imidazole moieties present on the CL reacts with either the carboxylic groups or hydroxyl groups of polysaccharides to form amide or ester cross-linkages respectively. The structure of the reactive molecule of the CL was confirmed by NMR spectra and mass spectroscopy.

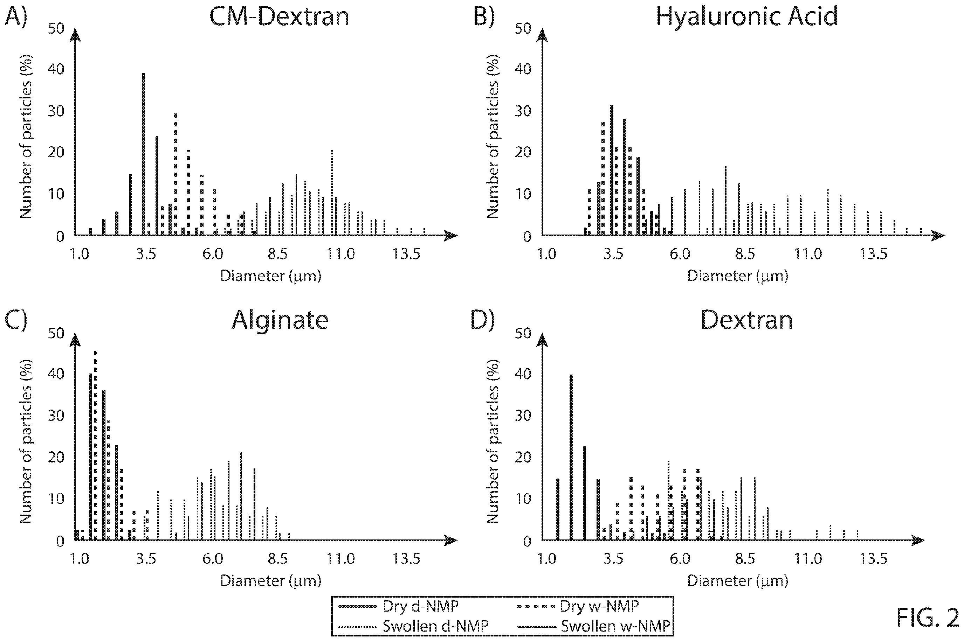

Table I summarizes some relevant outcomes of the synthesis process (i.e. NMP diameter, surface pore size, yield, swelling ratio) for each batch in this analysis. The NMP particles always show a highly porous outer surface, with pores ranging from 60 to 160 nm, as estimated from SEM images in the vacuum-dry state. As for the NMP diameter, SEM and confocal analysis deliver information about swollen and dry particles, allowing the computation of swelling ratio of NMP in water. The results suggest that the particles are quite uniform in size, either in dry or swollen state. The high swelling ratio values measured for any NMP ranged from 1.6 to 5.3 and indicated a high capability to adsorb water in all cases. The swelling capability of NMP is a telling ex-post measurement of capillary suction of a NMP microsponge, which is very relevant for drug upload, and is correlated to both the nanoporosity structure and the elastic response of the CL system. In particular, a discernible difference in the porosity was noticed for alginate based NMPs, exhibiting a remarkable capacity to swell up to 500 % in combination with the smaller pores. For drug delivery purposes, the optimal pore diameter (indicative of the capillarity structure for a given NMP diameter) could be seen as a trade-off between maximizing the effective water uptake and ensuring sufficient higher cut-off in the size of drugs or biomacromolecules to enter into the NMP. For a better comparison of NMP diameter results, the size distribution for each batch are shown in Figure 2.

**Table I**

| **Polysaccharide** | **Solvent for CL** | **Average Yield (%)** | **NMP Diameter** | | | **NMP Pores Diameter** |
|---|---|---|---|---|---|---|
| | | | **Dry (SEM)** | **Swollen** | **Swelling Ratio** | |
| CM-Dextran | DMSO | 97 | 3.5 ± 0.8 µm | 9.8 ± 1.7 µm | 2.8 | 0.10 ± 0.04 µm |
| | H₂O | 94 | 5.0 ± 0.1 µm | 9.6 ± 1.8 µm | 1.9 | 0.13 ± 0.05 µm |
| Hyaluronic Acid | DMSO | 98 | 4.0 ± 0.8 µm | 11.2 ± 1.8 µm | 2.8 | 0.16 ± 0.06 µm |
| | H₂O | 98 | 3.4 ± 0.7 µm | 7.3 ± 1.4 µm | 2.1 | 0.14 ± 0.05 µm |
| Alginate | DMSO | 98 | 1.4 ± 0.4 µm | 5.2 ± 1.6 µm | 3.5 | 0.06 ± 0.02 µm |
| | H₂O | 98 | 1.2 ± 0.5 µm | 6.3 ± 1.0 µm | 5.3 | 0.08 ± 0.03 µm |
| Dextran | DMSO | 96 | 2.4 ± 0.8 µm | 7.4 ± 1.8 µm | 3.0 | 0.11 ± 0.04 µm |
| | H₂O | 96 | 4.8 ± 1.0 µm | 7.5 ± 1.4 µm | 1.6 | 0.09 ± 0.04 µm |

In order to have a comparison, NMPs were produced starting from hyaluronic acid with an average MW of 800 kDa. All the above preparation conditions were repeated. These NMPs were measured to have an average diameter of 3.3 ± 0.9 µm in the absence of water and an average diameter of 4.2 ± 0.9 µm when swollen due to the presence of water. As can be immediate, the NMPs of this comparison example have a swelling ratio much lower than those of the hyaluronic acid NMPs of the invention.

From a synthesis stand-point, the reaction yields for each polysaccharide are consistently quite high, which is industrially relevant when considering that such values were observed also while scaling up the reaction.

Finally, the degradation of NMP under redox treatment was evaluated as it is an important parameter for applications entailing the slow release of the polysaccharide. As the CL bears a disulfide bridge, the inventors verified the ability of NMP to be deconstructed under redox treatment. RITC labelled NMPs were incubated in a solution of DTT 10 mM in PBS a reducing agent that could mimic the glutathione action in the biological environment. After 10 h incubation with DTT, approximately 80 % of NMPs was disassembled into soluble polysaccharide chains.

### NMP as platform for sequestration and release of proteins

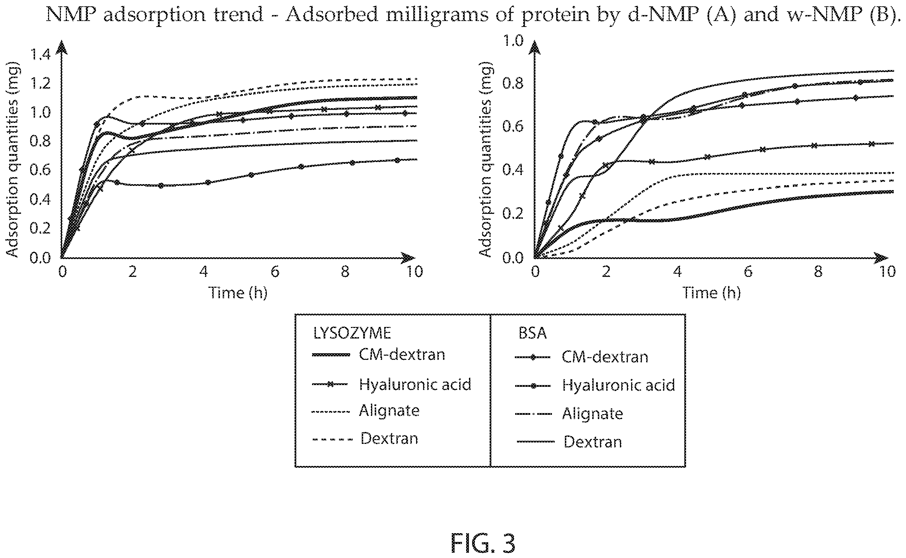

To demonstrate the marked capability of NMPs to sequestrate proteins from a medium at physiological conditions (pH 7.4), BSA and Lysozyme were chosen as two models of positively and negatively charged proteins respectively. In addition, while BSA is a relatively large protein (MW 65 kDa) and an isoelectric point (pI) of 5.3, Lysozyme is a small enzyme (MW 14 kDa) with pI of 11.4. Non-labelled NMPs were incubated in aqueous solutions of RITC labelled proteins for 24 h, the adsorption capacity was quantified after the centrifugation of NMP and analysis of the supernatant (Table II). The adsorption capacity of each NMP was evaluated when a plateau in the absorption curve was reached (Figure 3).

**Table II**

| | Adsorbed Lysozyme quantity (mg) after 24h | | Adsorbed BSA quantity (mg) after 24h | |
|---|---|---|---|---|
| | d-NMP | w-NMP | d-NMP | w-NMP |
| CM-Dextran | 1.099 | 0.441 | 0.999 | 0.850 |
| Hyaluronic acid | 1.155 | 0.553 | 0.814 | 0.894 |
| Alginate | 1.252 | 0.402 | 0.944 | 0.886 |
| Dextran | 1.225 | 0.432 | 0.868 | 0.913 |

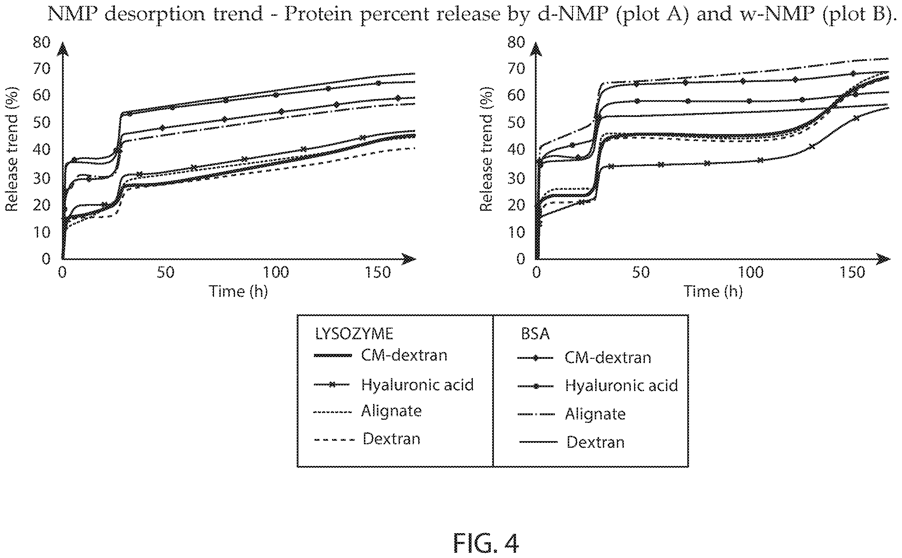

The release of proteins from loaded NMPs was evaluated next by replacing PBS solution and monitoring release via UV-vis spectra at different incubation times up to 180 h. The release curves of all NMPs are shown in Figure 4. Regardless the different distribution of the proteins inside the NMP the release curves showed a step-wise profile with two steps. The first step was completed within 24 h incubation with the medium, whereas the second step exhibited a burst followed by a prolonged and sustained release trend of proteins from NMPs lasting up to 250 h. Such a burst in release may be ascribed the loosely bound proteins on the NMP outer surface.

The comparison between loading and release efficiency for Lysozyme and BSA in Figure 3 and Figure 4 indicates the following:
(i) Proteins uptake of d-NMP and w-NMP varies between 1.2 - 0.8 and 0.4 -0.8 mg protein/mg polysaccharide respectively. Lysozyme is preferentially up-taken by d-NMP whereas serum albumin is equally adsorbed by both d-NMP and w-NMP. This indicates that the composite nanostructured template of d-NMP interacts more efficiently with different type of proteins.
(ii) On the other end, the efficiency of w-NMP (70 - 80 %) in releasing the adsorbed proteins is higher than d-NMP efficiency (40 - 70 %) .
(iii) The adsorption is nearly completed after 4h while the release typically occurs on a scale of days.

Overall these results demonstrate the capability of NMPs towards uploading proteins and highlight a characteristic asymmetry between adsorption and desorption. In fact, the strong capillary interactions that are responsible for the efficient protein-loading of NMPs is also the cause of slow release during discharge, opposing the osmotic gradient pull.

### Dependence of adsorption/release profiles vs. pH and protein distribution within the NMP

The release profile depends on a number of parameters and one of the most important of which for many biomedical applications (e.g. drug delivery and intra-articular slow-delivery) is the pH encountered by loaded NMP. Therefore, in addition to the above results related to physiological condition (PBS at pH 7.4, hereafter shortened as pH 7), it was designed an experiment to assess the dependence of both adsorption and release performance of the eight NMP batches at pH 5 and 11. Hence, the six PBS solutions at pH 5, 7 and 11 containing RITC-lysozyme or RITC-BSA (6 samples) were monitored for 24 hours at room temperature to ensure the stability of proteins loading and release.

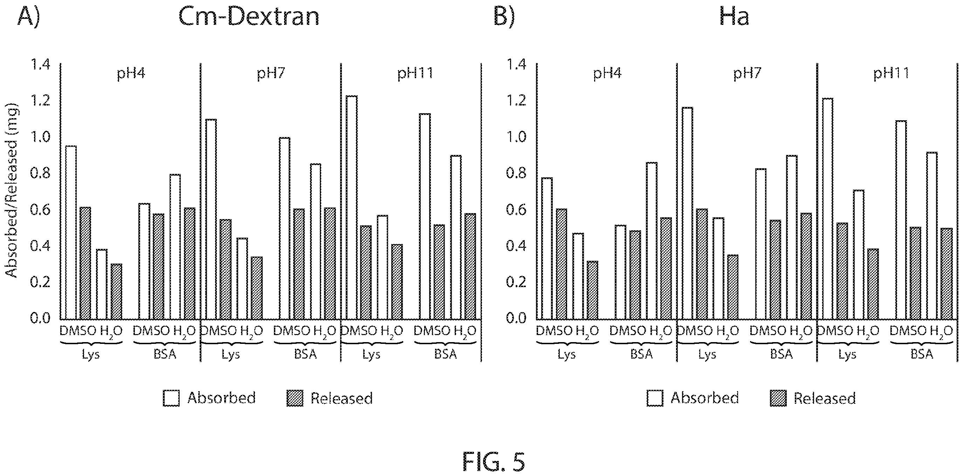

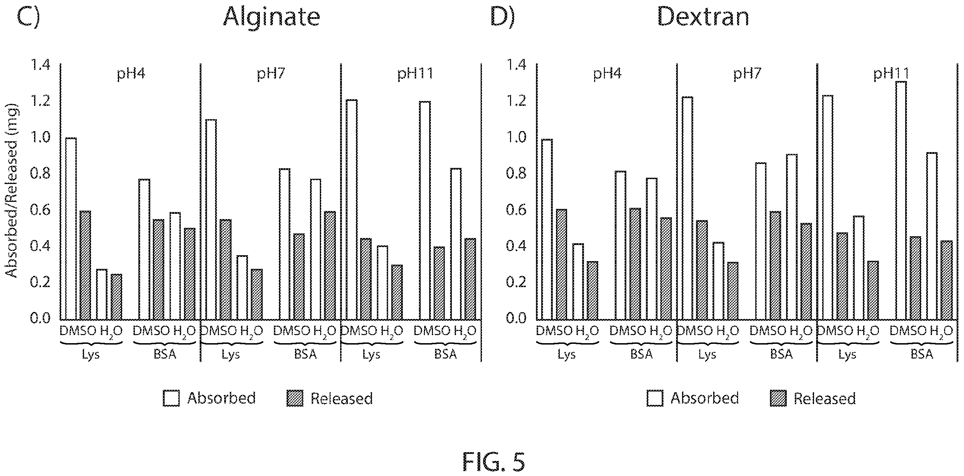

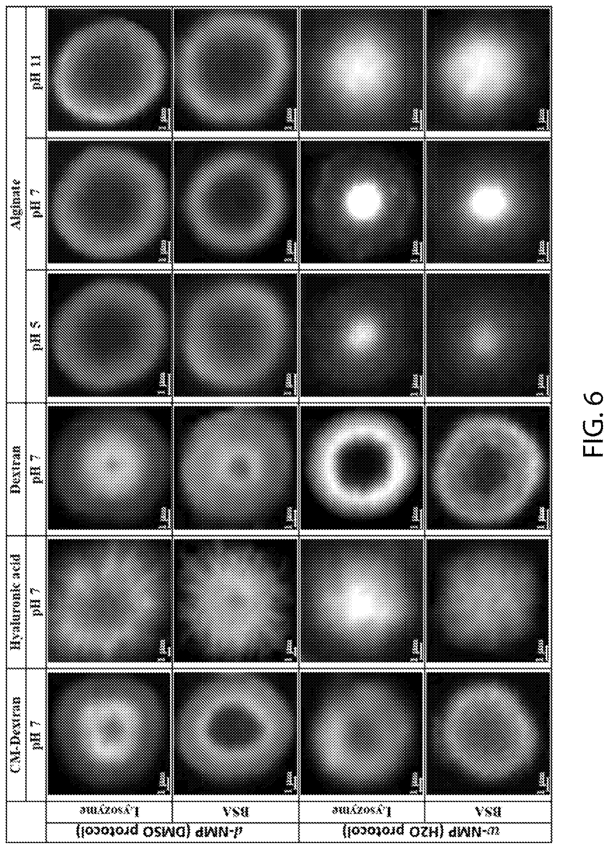

By the adsorption and desorption procedure described above, the measurement of the two plateau of the adsorption curve (i.e. the maximum of adsorption at 24 hours) and of release curve (after 150 hours) rendered the results in Figure 5 for each of sample in the experiment. As general findings emerging from this study:
(i) The absolute adsorption/release performance depends on the specific NMP polysaccharide (i.e. size, porosity etc., as discussed in Sec 3.1);
(ii) The adsorption and release rates tend to increase with the pH value (ref. also Figure 6), although this effect is more marked on lysozyme than BSA.

The actual uptake of the protein, apart from the gross value, depends also on how it penetrates and distribute within the NMP, which varies considerably between the 48 cases. Observations from confocal microscopy help appreciating this aspect by rendering the distribution of (RITC-labelled) protein content within each NMP type (Figure 6).

### Toxicity assays

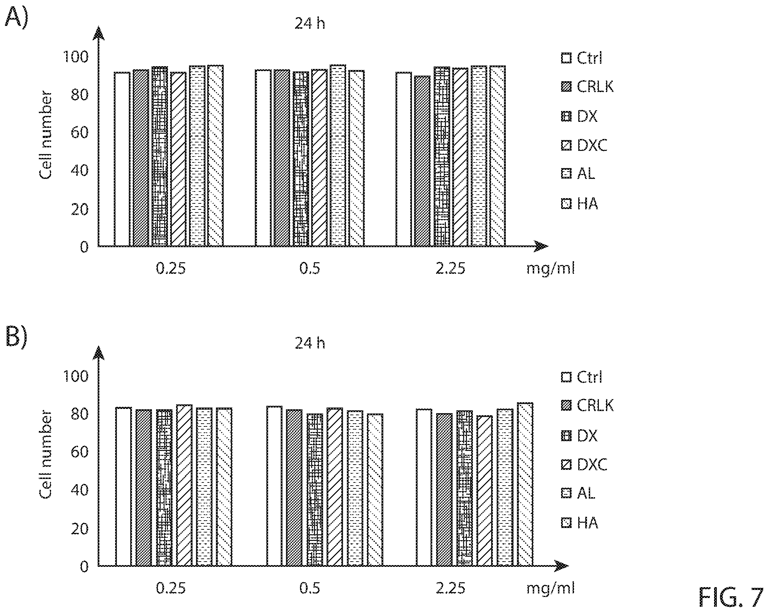

The toxicity of NMPs was assessed on T3T fibroblasts as survival cells in trypan blue test under several experimental conditions. Cells were grown in complete medium (Ctrl) or in complete medium supplemented with different concentrations ranging from 0.25 mg/mL to 2.25 mg/mL of all eight batches of NMPs, for 24 and 120 hours. As the CL exerts a vital role in the fabrication of NMPs, an additional control (CRLK) was performed adding the CL in complete medium without the polysaccharide. Results are reported in Figure 7 as percentage of living cells (n=3, p<0.05). No value was found significant compared to the relative controls, indicating lack of cytotoxicity of both CL and NMPs. This confirms that NMPs are indeed safe and non-toxic, as observed in our prior study. These results support a possible use of NMPs, as excipient, as well as for extracellular or intracellular release of different type of cargos.

### Study of cell interaction and uptake of NMPs

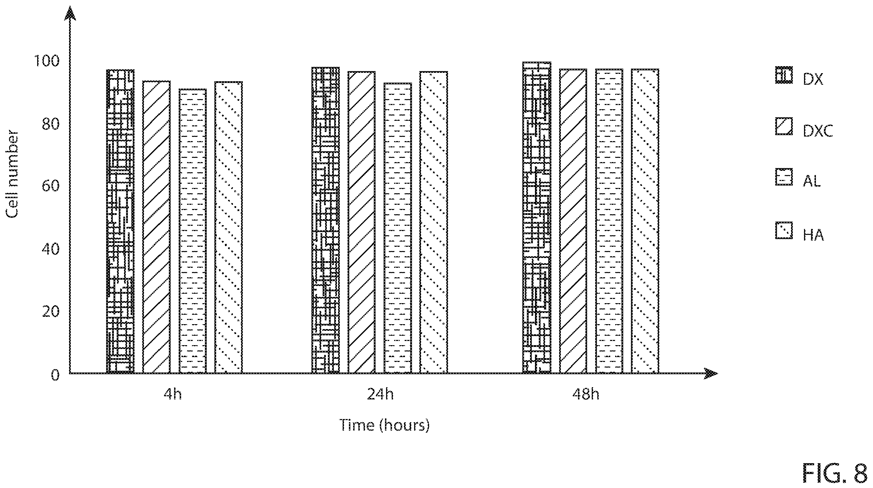

To analyse the interaction between NMPs to 3T3 cells, FITC-labelled NMPs were added to fibroblasts cultured in 24 multiwalls. The number of NMPs in each sample was quantified after 4, 24 and 48 h incubation, by counting via a Bürker-Türk counting chamber the unbound NMPs removed from suspension. Results in showed that a large fraction of NMPs readily associated with fibroblast within the first 4 h of incubation (Figure 8). However, associate NMPs can be either bound to cell membrane or internalized by endocytic processes.

In order to understand the nature of the uptake and determine whether NMPs localized inside or outside the cells, confocal microscopy analyses were performed. Fibroblasts were incubated with FITC-labeled NMPs (0.25 mg/ml) for 4, 24, 48 h. After incubation cells were stained with a red fluorescent marker WGA for highlighting plasma and Golgi/ER membrane structures. Numerous NMPs were visualized on treated cells by confocal microscopy imaging prolonging the incubation time up to 48 h (TOP panels) . All prepared NMPs appear localized (yellow signal) with the cell membrane marker (red signal) after 48 h incubation.

It is worth noting that NMP were found to non-specifically adsorb different dyes hence the localization study could be affected by this interference. Although the internalization process cannot be excluded for certain, the acquired confocal microscope micrographs convey that the NMPs are adherent to cell membrane and seem to localized on the outside of it, when examining all three projections.

Typically, microparticles with diameter longer than 0.5 µm have been known to enter cells via phagocytosis pathways. Generally, the phagocytosis pathway is restricted to specialized phagocytes, such as macrophages, monocytes and polymorphonuclear granulocytes. However, it has been shown that some cell types, such as fibroblasts, epithelial cells, and endothelial cells, can also internalize large particles. From confocal microscopy images it can be deduced that NMPs instead seem not be internalized and dock on the outside of the cell membrane. Overall, these studies show the effective interaction and internalization of NMP with fibroblast without compromising their proliferation.

From the above description is evident that the method of the invention allows to prepare NMPs in a very safe, easy and effective way, and that the produced NMPs show an efficient loading of BSA and lysozyme along with the sustained release of the two proteins.

In addition, the demonstrate adsorption-release performance on pH bears implications in medical applications and represents a possible way of tweaking the release profile.

Finally, it is important to underline that NMPs are not cytotoxic but markedly biocompatible.

In conclusion, the method and the nanoporous microsponge particles of the invention offer an innovative platform for biomedical and clinical applications.

## Claims

1. A method to produce nanoporous microsponge particles useful as carriers for drug delivery; said method comprising a cross-linking/precipitation step wherein a cross-linking agent reacts with hyaluronic acid having nucleophile functional groups which react with said cross-linking agent; said a cross-linking agent being prepared by a reaction between 1,1'-carbonyldiimidazole (CDI) and a diamine compound; said method being **characterized in that** said hyaluronic acid has an average molecular weight ranging from 10KDa to 200KDa.

2. A method according to claim 1, **characterised in that** said hyaluronic acid has an average molecular weight ranging from 20KDa to 150KDa.

3. A method according to claim 1 or 2, **characterised in that** said hyaluronic acid is used at a concentration ranging from 0.5 to 1.5 g/dL.

4. A method according to claim 1 or 2, **characterised in that** said hyaluronic acid is used at a concentration ranging from 0.8 to 1.0 g/dL.

5. A method according to anyone of the previous claims, **characterised in that** said cross-linking agent is used at a concentration ranging from 0.05 to 0.15 M.

6. A method according to anyone of the claims 1 to 4, **characterised in that** said cross-linking agent is used at a concentration ranging from 0.05 to 0.10 M.

7. A method according to anyone of the previous claims, **characterised in that** said diamine compound is Cystamine dihydrochloride.

8. Nanoporous microsponge particles prepared by the method according to anyone of the previous claims and **characterized by** having an average diameter ranging from 1.0 to 15.0 µm and an average size of pores ranging from 10 to 400 nm.

9. Nanoporous microsponge particles according to claim 8, **characterized by** having a swelling ratio higher than 1.5.

10. Nanoporous microsponge particles according to claim 8 or 9 for use as carriers for drug delivery.

11. Nanoporous microsponge particles according to claim 8 or 9 for use as a medicament.

## Patentansprüche

1. Verfahren zum Produzieren von nanoporösen Mikroschwammpartikeln, die als Träger zur Medikamentenabgabe nützlich sind; wobei das Verfahren einen Vernetzungs-/Ausfällungs-Schritt umfasst, in dem ein Vernetzungsmittel mit Hyaluronsäure, die nucleophile funktionelle Gruppen aufweist, die mit dem Vernetzungsmittel reagieren, reagiert; wobei das Vernetzungsmittel durch eine Reaktion zwischen 1,1'-Carbonyldiimidazol (CDI) und einer Diaminverbindung hergestellt wird und das Verfahren **dadurch gekennzeichnet ist, dass** die Hyaluronsäure ein durchschnittliches Molekulargewicht im Bereich von 10 KDa bis 200 KDa besitzt.

2. Verfahren nach Anspruch 1, **dadurch gekennzeichnet, dass** die Hyaluronsäure ein durchschnittliches Molekulargewicht im Bereich von 20 KDa bis 150 KDa besitzt.

3. Verfahren nach Anspruch 1 oder 2, **dadurch gekennzeichnet, dass** die Hyaluronsäure in einer Konzentration im Bereich von 0,5 bis 1,5 g/dL verwendet wird.

4. Verfahren nach Anspruch 1 oder 2, **dadurch gekennzeichnet, dass** die Hyaluronsäure in einer Konzentration im Bereich von 0,8 bis 1,0 g/dL verwendet wird.

5. Verfahren nach einem der vorhergehenden Ansprüche, **dadurch gekennzeichnet, dass** das Vernetzungsmittel in einer Konzentration im Bereich von 0,05 bis 0,15 M verwendet wird.

6. Verfahren nach einem der Ansprüche 1 bis 4, **dadurch gekennzeichnet, dass** das Vernetzungsmittel in einer Konzentration im Bereich von 0,05 bis 0,10 M verwendet wird.

7. Verfahren nach einem der vorhergehenden Ansprüche, **dadurch gekennzeichnet, dass** die Diaminverbindung Cystamindihydrochlorid ist.

8. Nanoporöse Mikroschwammpartikel hergestellt gemäß dem Verfahren nach einem der vorhergehenden Ansprüche und **gekennzeichnet durch** einen durchschnittlichen Durchmesser im Bereich von 1,0 bis 15,0 µm und eine durchschnittliche Größe von Poren im Bereich von 10 bis 400 nm.

9. Nanoporöse Mikroschwammpartikel nach Anspruch 8, **gekennzeichnet durch** ein Quellverhältnisses größer als 1,5.

10. Nanoporöse Mikroschwammpartikel nach Anspruch 8 oder 9 zur Verwendung als Träger bei der Medikamentenverabreichung.

11. Nanoporöse Mikroschwammpartikel nach Anspruch 8 oder 9 zur Verwendung als ein Medikament.

## Revendications

1. Procédé de production de particules de micro-éponges nanoporeuses pouvant être utilisées comme supports destinés à l'administration de médicaments ; ledit procédé comprenant une étape de réticulation/précipitation dans laquelle un agent de réticulation réagit avec de l'acide hyaluronique ayant des groupes fonctionnels nucléophiles qui réagissent avec ledit agent de réticulation ; ledit agent de réticulation est préparé à l'aide d'une réaction entre le 1,1'-carbonyldiimidazole (CDI) et un composé diaminé ; ledit procédé étant **caractérisée en ce que** que ledit acide hyaluronique présente une masse moléculaire moyenne allant de 10 KDa à 200 KDa.

2. Procédé selon la revendication 1, **caractérisé en ce que** ledit acide hyaluronique présente une masse moléculaire moyenne allant de 20 KDa à 150 KDa.

3. Procédé selon la revendication 1 ou 2, **caractérisé en ce que** ledit acide hyaluronique est utilisé à une concentration allant de 0,5 à 1,5 g/dL.

4. Procédé selon la revendication 1 ou 2, **caractérisé en ce que** ledit acide hyaluronique est utilisé à une concentration allant de 0,8 à 1,0 g/dL.

5. Procédé selon l'une quelconque des revendications précédentes, **caractérisé en ce que** ledit agent de réticulation est utilisé à une concentration allant de 0,05 à 0,15 M.

6. Procédé selon l'une des revendications 1 à 4, **caractérisé en ce que** l'agent de réticulation est utilisé à une concentration allant de 0,05 à 0,10 M.

7. Procédé selon l'une quelconque des revendications précédentes, **caractérisé en ce que** le composé diaminé est le dihydrochlorure de cystamine.

8. Particules de micro-éponges nanoporeuses préparées à l'aide du procédé selon l'une quelconque des revendications précédentes et **caractérisées en ce qu'**elles présentent un diamètre moyen allant de 1,0 à 15,0 µm et une taille moyenne des pores allant de 10 à 400 nm.

9. Particules de micro-éponges nanoporeuses selon la revendication 8, **caractérisées par** un taux de gonflement supérieur à 1,5.

10. Particules de micro-éponges nanoporeuses selon la revendication 8 ou 9 destinées à être utilisées en tant que supports pour l'administration de médicaments.

11. Particules de micro-éponges nanoporeuses selon la revendication 8 ou 9 destinées à être utilisées en tant que médicament.
